# EUROPEAN PATENT SPECIFICATION

(11) **EP 0 904 295 B1**
(45) Date of publication and mention of the grant of the patent: **10.08.2005**
(21) Application number: 97923892.0
(22) Date of filing: 14.05.1997
(51) Int. Cl.: C07K 14/785, G01N 30/94

(54) **PROCESS FOR SEPARATING AND ANALYZING HYDROPHOBIC PROTEINS USING THIN LAYER CHROMATOGRAPHY**
VERFAHREN ZUR TRENNUNG UND BESTIMMUNG VON HYDROPHOBEN PROTEINEN DURCH DÜNNSCHICHTCHROMATOGRAPHIE
PROCEDE DE SEPARATION ET D'ANALYSE DE PROTEINES HYDROPHOBES PAR CHROMATOGRAPHIE EN COUCHE MINCE

(30) Priority: 15.05.1996 DE 19619576; 04.06.1996 EP 96108905
(43) Date of publication of application: 31.03.1999
(73) Proprietor: ALTANA Pharma AG, 78467 Konstanz (DE)
(72) Inventor: ISE, Wolfgang, D-78462 Konstanz (DE); NAVE, Rüdiger, D-78464 Konstanz (DE); STEINHILBER, Wolfram, D-78333 Stockach (DE)
(74) Representative: Rupp, Herbert, Dr.
(86) International application number: PCT/EP1997/002463
(87) International publication number: WO 1997/043313

(56) References cited:
- EP-A- 0 652 227
- US-A- 4 338 301
- US-A- 5 227 308
- US-A- 5 258 496
- US-A- 5 443 989
- US-A- 5 455 227
- I. RUSTENBECK ET AL: "quantitation of hexadecylphosphiocholine by high-performance thin-layer chromatography with densitometry" JOURNAL OF CHROMATOGRAPHY., vol. 525, 1990, AMSTERDAM NL, pages 85-91, XP002037277
- K. KORTE ET AL.: "phospholipid and neutral lipid separation by one-dimensional thin-layer chromatography" JOURNAL OF CHROMATOGRAPHY., vol. 232, 1982, AMSTERDAM NL, page 47-53 XP002037278
- A.M. GILFILLAN ET AL.: "single plate separation of lung phospholipids including disaturated phosphatidylcholine" JOURNAL OF LIPID RESEARCH, vol. 24, no. 9, September 1983, page 1651-1656 XP002037279
- BENAJIA A. ET AL: 'PRESENCE OF FREE HYDROPHOBIC PEPTIDES IN THE BRUSH BORDER AND BASOLATERAL MEMBRANES OF PIG ENTEROCYTES' BIOCHIMICA ET BIOPHYSICA ACTA vol. 687, 1982, pages 1 - 7
- ASCH ET AL: 'Covalent binding of lipids to cytoskeletal proteins of mouse mammary epithelial cells' CELL BIOLOGY INTERNATIONAL REPORTS vol. 16, no. 1, 1992, pages 83 - 91
- KERESE I.: 'Methods of protein analysis', 1984, ELLIS HORWOOD LIMITED, ENGLAND

## Description

### Technical field

The invention relates to a process for the detection and determination of strongly hydrophobic proteins, protein fragments and modifications, and of strongly hydrophobic peptides.

### Prior art

The quantification of hydrophobic proteins is not possible or is only inadequately possible using the conventionally customary techniques [Ellis Horwood Limited, Publishess and Akademia kiado, Budapest 1984: METHODS OF PROTEIN ANALYSIS, chapter 5, pages 206-270 'Thin-layer chromatography of protein, peptides and amino-acide']. The methods customary for the separation of hydrophilic proteins, such as, for example, Western blotting are often only applicable to hydrophobic proteins to a limited extent, because on the one hand the separation on the SDS gel is inadequate and on the other hand the transfer of the proteins to the customary membranes can be described as at most semiquantitative. A separation of slightly modified proteins (e.g. methyl esters) cannot be effected by means of gel electrophoresis. The use of immunological methods, such as, for example, ELISA is very problematical, because as a rule this can only be carried out in aqueous systems. Organic solvents may react with the material of the microtiter plates and render them unusable. The analytes to be quantified are usually present only in traces. Often, straight hydrophobic proteins are additionally associated with other lipophilic substances (e.g. lipids), which makes a quantification according to conventional methods impossible. In ELISA, the components are not separated from one another.

Benajiba et al. [Biochimica et Biophysica Acta, Vol. 687, 1982, pages 1-7, A. Benajiba et al. 'Presence of free hydrophobic peptides in brush border and basolateral membranes of pig enterocytes'] refers to separation of short, hydrophobic peptides from membrane lipids by thin layer chromatography (TLC) using chloroform/methanol/water as developer. Peptides are stained with 0,2% Ninhydrin in acetat.

Asch et al. [Cell Biology International Reposts, Vol. 16, No.1, 1992, pages 83-91, H.L. Asch et al. 'Covalent binding of lipids to cytoskeletal proteins of mouse mammary epithelial cells'] refers to the analysis of cytoskelatal proteins, whereby the proteins, are subjected to a specific procedure to extract bound lipids comprising TLC. The extraction efficacy of the procedure is monitosed by radioactivity testing using ³H-palmitate labeling.

### Description of the invention

The aim of the invention is the provision of a process which allows hydrophobic proteins, protein fragments and modifications and also strongly hydrophobic peptides to be separated and to be quantified.

A further aim is to make available a process which makes possible the determination of, for example, proteins which are slightly modified by acetylation or oxidation.

It is a further aim to indicate such a process which is suitable, in particular, for the determination of hydrophobic host-cell protein impurities (HCP) in the biotechnological production of hydrophobic proteins, such as, for example, the extremely hydrophobic lung surfactant protein SP-C.

It has now been found that these aims are achieved by thin-layer chromatographic separation of the protein mixture dissolved in an organic medium and immunological detection of the separated proteins.

The object of the invention is therefore a process for the separation and quantification of strongly hydrophobic proteins, protein fragments and modifications and also of strongly hydrophobic peptides, which comprises dissolving the samples to be investigated in an organic medium, subjecting them to thin-layer chromatography and rendering the hydrophobic proteins visible immunologically.

Further objects are clear from the subclaims.

It has surprisingly been found that for the thin-layer chromatographic separation step, procedures and materials known per se which were adapted to the hydrophobic properties of the proteins to be separated lead to the aim. Possible chromatography plates are all plates whose coating is suitable for the separation of hydrophobic mixtures in organic media. Of these, those which prove particularly suitable are the HPTLC plates marketed by Merck Darmstadt under the trade name Diol, which have a modified silica matrix. For hydrophobic proteins, suitable mobile phases for thin-layer chromatography are organic solvents and solvent mixtures, e.g. of chloroform and methanol. Mixtures of nonpolar and polar solvents are particularly expedient, possible nonpolar solvents, in particular, being chloroform, methylene chloride and toluene and polar solvents being short-chain alcohols, in particular methanol, ethanol and isopropanol.

The application of the samples to the plates and the separation procedure are carried out in a customary manner, for example by means of commercially available automatic equipment.

For preparation for immunological detection, the plates are dried after thin-layer chromatographic separation. For the saturation of nonspecific binding sites, the plates are incubated with a suitable blocking solution, e.g. gelatin or proteins. The plates are then incubated with the primary antibody. If this does not carry a label, detection can be carried out with the aid of a labeled second antibody. For detection, all commercially available detection processes can be used. After removal of excess first antibody by washing, incubation is carried out with a labeled second antibody. After washing, the labeled antibodies are detected. They are visualized in a customary manner, e.g. by addition of luminol and hydrogen peroxide, for example with the aid of the ECL (Enhanced Chemiluminescence) detection process of Amersham Buchler, which is very sensitive. In the purity analysis, with a view to slight chemical modifications, the substances can be detected on the TLC plates directly after separation also using customary protein staining reagents.

In the following text, the invention is described by example with the aid of a process for the determination of the host-cell protein impurities in a biotechnological process for the preparation of r-SP-C by means of E. coli.

### Example

### 1. Obtainment of HCP for immunization purposes

In agreement with procedures known from the literature and with relevant official regulations, an antigen fraction from the final phase of the downstream process was sought. Therefore, for the immunization, HCPs were withdrawn from a fermentation phase in which the r-SP-C is pure to 80 to 90%. For the removal of the HCP antigen fraction, 60 g of inclusion bodies isolated by filtration an/or centrifugation from a 10 l blank fermentation were used after lyophilisation for about 96 hours.

### 2. Preparation of antisera

1 mg of HCP, dissolved in 95% strength isopropanol of pH 2, was dried in a vacuum concentrator (speedvac®), resuspended in 0.5 ml of phosphate-buffered saline, mixed with 0.5 ml of adjuvant (ABM-S for the base immunization and ABM-N for the booster injections) and in each case injected subcutaneously in an amount of 1 mg/rabbit. The immunization scheme was carried out according to standard protocols: after the primary immunization, booster injections were carried out every 4 weeks up to six times. Taking of blood was in each case carried out 10 days after the last injection in order to monitor the development of the titer. As soon as the titer was satisfactory, 50 ml of blood were taken and serum was prepared according to standard processes.

### 3. Determination of the titer

For determination of the titer, the individual sera at various dilutions were analyzed by means of the new method described as immuno-thin-layer chromatography (immuno-TLC). Dilutions of 1:5,000, 1:10,000, 1:20,000 and 1:50,000 were used in order to analyze 4, 16, 62.5 and 250 ng of HCP. At a dilution of 1:10,000, all rabbit antibodies investigated recognized HCP components in proportion to the amount of total protein. Antisera having a similar titer were pooled and reanalyzed. The serum was characterized according to standard processes and stored in aliquots at -20°C.

### 4. Sample preparation and thin-layer chromatography (TLC)

The samples to be analyzed were dried in a vacuum concentrator and dissolved in 20 to 200 µl of CHCl₃/MeOH. For thin-layer chromatography, HPTLC plates having a modified silica matrix, such as are marketed by Merck Darmstadt under the trade name Diol, were used. The application of the samples to the HPTLC plates was carried out automatically using a Lingomat IV (Camag, Berlin). After sample application, the plates were air-dried and subjected to chromatography using a CHCl₃/MeOH mixture [CHCl₃/MeOH/25% strength NH₄OH/H₂O = 32.5/15/1/2 (ratio by volume)] as the liquid phase. After chromatography, the plates were dried.

### 5. Immunostaining of HCP with anti-HCP antibodies

For the saturation of nonspecific binding sites, the dried HPTLC plates were incubated for 4 hours with 3% strength fish gelatin in PBS which contained 150 mM NaCl, 12 mM Na₂HPO₄ and 3 mM NaH₂PO₄ (pH 7.4). The plates were then incubated overnight and lightly shaken in the presence of the primary antibody, usually at a dilution of 1:10,000. Unbound antibodies were removed by washing several times with TBS/T from 4 mM tris HCl, 100 mM NaCl, 0.05% Tween 20 (pH 7.4). For hybridization with the primary antibody, the plates were incubated for 2 hours with horseradish peroxidase-conjugated secondary antibody at a dilution of 1:80,000 in the TBS/T. Unbound antibodies were removed as described above by washing the plates several times with TBS/T. Immunoreactive complexes were visualized using an ECL detection system from Amersham Buchler. The plates were incubated for 20 to 60 seconds with an X-ray film (Hyperfilm Amersham).

### 6. Staining and immunostaining of SP-C

In order to detect modifications of SP-C (e.g. methyl ester at the C-terminus and methionine sulfoxide), the protein forms separated by means of TLC can be detected by means of staining with Ponceau S. A more sensitive possibility is the use of SP-C antibodies and of the method indicated above in analogy to HCP determination.

### 7. Video-imaging of the X-ray films and computer analysis

For the quantification of the immune complexes, the X-ray films were digitalized using a video-imager (Cybertech, Berlin, Germany). The signal intensities on the X-ray films were analyzed by computer analysis using Wincam software (Cybertech).

### 8. Quantification of HCP

For the determination of HCP in r-SP-C samples, aliquots were analyzed pure or after addition of 2.5, 5.0 or 10.0 ng of HCP. The amounts of HCP in the individual samples were determined by linear regression calculations. Percentages were determined by means of the formula % = (ng of HCP x 100)/ng of r-SP-C.

In order to quantify small amounts of HCP in r-SP-C, it is necessary to separate the majority of the target protein from the small amount of HCP. This is effected by thin-layer chromatography. The detection limit for HCP was analyzed by mixing 5 µg of highly pure r-SP-C with ng amounts of HCP. Under these conditions, 0.125 ng of HCP was the lowest amount which it was possible to differentiate from the endogenous HCP content of r-SP-C. The amount of the sample which can be analyzed by immuno-TLC depends on the HCP content. For r-SP-C samples which contain less than 0.1% of HCP, 1 to 5 µg of r-SP-C are analyzed. Since it is possible to separate amounts of r-SP-C up to 20 µg by thin-layer chromatography and the quantification limit is 1 ng on luminography (when using 5 µg of added r-SP-C), it was possible to detect HCP theoretically down to 0.005%. The detection of 0.002% HCP appears to be a reliable quantification limit under standard conditions.

## Claims

1. A process for the separation, determination and quantification of a hydrophobic host-cell protein impurity in a biotechnological product of hydrophobic proteins, which comprises dissolving a sample to be investigated in an organic medium, subjecting it to thin-layer chromatography (TLC) and detecting the hydrophobic host-cell protein impurity by means of immunological methods directly on the TLC plate and quantifying the host-cell protein.

2. A process as claimed in claim 1 wherein the organic medium is a mixture of chloroform and methanol.

3. A process as claimed in claim 1 wherein the quantification is effected by an immunological method.

4. A process as claimed in claim 3 wherein the immunological method comprises binding the hydrophobic host-cell protein impurity to a labeled antibody.

5. A process as claimed in claim 4 which comprises visualizing and quantifying immunoreactive complexes.

6. A process as claimed in claim 5 wherein the visualizing and quantifying are effected with the aid of enhanced chemiluminescence.

7. A process as claimed in claim 1 which comprises effecting the thin layer chromatography on coated plates having a modified silico matrix.

8. A process as claimed in claim 1 wherein the organic medium is a mixture of solvents in which the hydrophobic host-cell protein impurity is soluble.

## Patentansprüche

1. Verfahren zur Trennung, Bestimmung und Quantifizierung einer hydrophoben Wirtszellenproteinverunreinigung in einem biotechnologischen Produkt von hydrophoben Proteinen, **dadurch gekennzeichnet, daß** die zu untersuchenden Proben in einem organischen Medium gelöst und einer Dünnschichtchromatographie (DC) unterzogen werden und die hydrophobe Wirtszellenproteinverunreinigung durch immunologische Methoden direkt auf der DC-Platte detektiert werden und das Wirtszellenprotein quantifiziert wird.

2. Verfahren nach Anspruch 1, **dadurch gekennzeichnet, daß** es sich bei dem organischen Medium um eine Mischung aus Chloroform und Methanol handelt.

3. Verfahren nach Anspruch 1, **dadurch gekennzeichnet, daß** die Quantifizierung durch eine immunologische Methode erfolgt.

4. Verfahren nach Anspruch 3, **dadurch gekennzeichnet, daß** die immunologische Methode die Bindung der hydrophoben Wirtszellenproteinverunreinigung an einen markierten Antikörper umfaßt.

5. Verfahren nach Anspruch 4, **dadurch gekennzeichnet, daß** man immunreaktive Komplexe sichtbar macht und quantifiziert.

6. Verfahren nach Anspruch 5, **dadurch gekennzeichnet, daß** das Sichtbarmachen und die Quantifizierung mit Hilfe von verstärkter Chemilumineszenz (enhanced chemiluminescence) erfolgt.

7. Verfahren nach Anspruch 1, **dadurch gekennzeichnet, daß** die Dünnschichtchromatographie auf beschichteten Platten mit einer modifizierten Silica-Matrix erfolgt.

8. Verfahren nach Anspruch 1, **dadurch gekennzeichnet, daß** es sich bei dem organischen Medium um ein Lösungsmittelgemisch handelt, in dem die hydrophobe Wirtszellenproteinverunreinigung löslich ist.

## Revendications

1. Processus pour la séparation, la détermination et la quantification d'une impureté protéique hydrophobe d'une cellule hôte existant dans un produit de biotechnologie de protéines hydrophobes, qui comprend les étapes consistant à : dissoudre un échantillon à étudier dans un milieu organique ; le soumettre à une chromatographie sur couche mince (TLC) ; détecter l'impureté protéique hydrophobe d'une cellule hôte, au moyen de procédés immunologiques, directement sur la plaque de TLC ; et quantifier la protéine de la cellule hôte.

2. Processus selon la revendication 1, dans lequel le milieu organique est un mélange de chloroforme et de méthanol.

3. Processus selon la revendication 1, dans lequel la quantification est effectuée par un procédé immunologique.

4. Processus selon la revendication 3, dans lequel le procédé immunologique comprend la liaison de l'impureté protéique hydrophobe d'une cellule hôte à un anticorps marqué.

5. Processus selon la revendication 4, qui comprend la visualisation et la quantification de complexes immuno-réactifs.

6. Processus selon la revendication 5, dans lequel la visualisation et la quantification sont effectuées à l'aide d'une chimiluminescence amplifiée.

7. Processus selon la revendication 1, qui comprend l'étape consistant à effectuer la chromatographie sur couche mince sur des plaques enrobées ayant une matrice en silice modifiée.

8. Processus selon la revendication 1, dans lequel le milieu organique est un mélange de solvants, dans lequel est soluble l'impureté protéique hydrophobe d'une cellule hôte.
